# EUROPEAN PATENT SPECIFICATION

(11) **EP 1 073 492 B1**
(45) Date of publication and mention of the grant of the patent: **22.08.2007**
(21) Application number: 99919933.4
(22) Date of filing: 21.04.1999
(51) Int. Cl.: A61M 16/00, A62B 7/00, A62B 18/08, F23D 11/00, F23D 14/00, F24J 3/00

(54) **DISPOSABLE ACTIVE HUMIDIFIER FOR THE MECHANICAL VENTILATION OF A PATIENT**
AKTIVER EINWEGBEFEUCHTER FÜR DIE KÜNSTLICHE BEATMUNG EINES PATIENTEN
HUMIDIFICATEUR ACTIF JETABLE DESTINE A LA VENTILATION ARTIFICIELLE D'UN PATIENT

(30) Priority: 22.04.1998 IT MI980862
(43) Date of publication of application: 07.02.2001
(73) Proprietor: MALLINCKRODT, INC., St. Louis Missouri 63134 (US)
(72) Inventor: GIBERTONI, Lucio, I-41037 Mirandola (IT)
(74) Representative: Carpmaels & Ransford
(86) International application number: PCT/US1999/008699
(87) International publication number: WO 1999/053985

(56) References cited:
- WO-A-96/20747
- US-A- 3 927 981
- US-A- 4 086 305
- US-A- 4 381 267
- US-A- 4 449 992
- US-A- 5 035 236
- US-A- 5 062 145
- US-A- 5 192 499
- US-A- 5 435 298

## Description

The present invention relates to a disposable active humidifier for the mechanical ventilation of a patient.

It is known that the mechanical ventilation of a patient uses dry gases which are humidified before being inspired by the patient. Most commercially available systems are based on the principle of making the gas flow directly over the water contained in a heated container.

The conventional solution has a drawback which is constituted by the relatively high volume of the cartridge, which constitutes an additional bulk; it should be noted that it is very important to reduce bulk, since in mechanical ventilation, in which the pressure of the ventilator expands the lungs of the patient to ventilate him, an extra compressible space is certainly a negative factor. A document disclosing the features of the preamble of claim 1 is WO 96/20747.

The aim of the invention is indeed to eliminate the drawbacks mentioned above, which are typical of conventional systems (bacterial contamination and high compressible volume), by providing a disposable active humidifier for the mechanical ventilation of a patient which has a very small bulk and in particular constitutes an integrated segment in the ventilation circuit and in practice therefore does not increase the volume of compressible air.

Within the scope of this aim, a particular object of the invention is to provide a disposable active humidifier which can be prepared in a sterile package which can be connected only once to the patient without requiring further handling with the risk of bacterial contamination.

Another object of the present invention is to provide an active humidifier in which the water remains confined within the cartridge with a continuous supply which in practice forms a closed circuit with no need for connection to the outside.

Another object of the present invention is to provide a disposable active humidifier which, by virtue of its particular constructive characteristics, is capable of giving the greatest assurances of reliability and safety in use.

This aim, these objects and others which will become apparent hereinafter are achieved by a disposable active humidifier for the mechanical ventilation of a patient, according to the invention as defined in claim 1.

Further characteristics and advantages will become apparent from the description of a preferred but not exclusive embodiment.

The disposable active humidifier for the mechanical ventilation of a. patient comprises a cartridge which internally forms a humidification chamber which is delimited by an inlet and by an outlet which are formed on respective caps which are arranged mutually opposite at the axial ends of the humidification chamber.

The cartridge is externally provided with a heat exchange surface, advantageously constituted by a tubular aluminum casing, which delimits an interspace formed by a hydrophobic membrane which delimits the humidification chamber.

Inside the humidification chamber there is a diffuser which is meant to direct the incoming air stream so that it flows over the membrane, so as to facilitate the transfer of humidity through the hydrophobic membrane that delimits the interspace, inside which water is introduced by means of an inlet, to which it is possible to connect a simple bag or bottle which continuously introduces the water into the interspace so that by means of the hydrophobic membrane it is possible to ensure the required degree of humidity of the air.

It should be added that the diffuser advantageously has protrusions shaped like a conical inclined surface, which facilitate the conveyance of the air stream against the membrane, which provides a barrier against bacteria, so that the system can be supplied with ordinary distilled water, thus reducing the operating costs.

The cartridge can be easily used in a ventilation circuit in which there is a heater which forms the seat for accommodating the external casing of the cartridge.

From the above description it is thus evident that the invention achieves the intended aim and objects, and in particular the fact is stressed that the particular structure that has been used allows to provide a disposable active humidifier which in practice does not increase the volume of air present in the ventilation circuit, since it provides a simple segment which is interposed in the ventilation circuit itself.

Furthermore, the new active humidifier is based on an innovative principle which is different from the other systems that are commercially available, since its humidification principle is based on the vaporization of water instead of on flowing over water present in a heated container.

The present system uses a humidifier cartridge which is integrated in the temperature-controlled ventilation circuit and which by vaporization charges the dry gases on the inspiratory line of the patient with humidity.

By virtue of the PVC temperature-controlled circuit in which the heating resistor is embedded in the external reinforcement spiral, the humidity released by the humidifier cartridge does not condense along the inspiratory line but is transferred in full to the patient.

The cartridge of the humidifier is constituted by an external body made of metal (aluminum) which acts as interface with the heating element of the humidifier. Inside the cartridge there is a hydrophobic membrane which provides the interface between the liquid and the vapor phase of the inspiratory line.

The hydrophobic membrane allows the passage of the vapor that forms as a consequence of the heating of the water contained in the cartridge,

The system can be supplied with ordinary sterile/double-distilled water**.**

The innovation of the system is that it uses a cartridge which is integrated in the circuit and is preassembled to the ventilator with the connections provided.

Differently from flow-over humidification systems, the present system eliminates the contact of the supply water with the outside environment, avoiding the risk of exogenous contamination of the patient and keeping the system dry without forming condensate in the ventilation circuit.

The invention thus conceived is susceptible of numerous modifications and variations.

All the details may furthermore be replaced with other technically equivalent elements.

In practice, the materials used, as well as the contingent shapes and the dimensions, may be any according to the requirements.

## Claims

1. A disposable active humidifier for the mechanical ventilation of a patient, the humidifier comprising a cartridge which forms a humidification chamber which is delimited by an inlet and by an outlet, the cartridge being adapted to be interposed in a ventilation circuit, and said cartridge having an interspace which is externally delimited by a heat exchange surface and internally delimited by a hydrophobic membrane which surrounds said humidification chamber, wherein a humidification fluid is introducible into said interspace such that the humidification fluid, once introduced into the cartridge, remains confined within the cartridge unless vaporising therefrom through the hydrophobic membrane, the cartridge being externally provided with said heat exchange surface in the form of an external tubular casing, which, in use, acts as an interface with a heating element for heating the humidification fluid within the humidifier, wherein said humidification chamber has a substantially cylindrical shape and, in use, forms a segment of said ventilation circuit, **characterised in that** said outlet and said inlet are formed by respective caps which axially delimit said interspace and said humidification chamber.

2. A disposable active humidifier according to claim 1 **characterised in that** it axially comprises, inside said humidification chamber, a diffuser which is adapted to direct the air stream of the ventilation circuit against said hydrophobic membrane.

3. A disposable active humidifier according to any one of the preceding claims, **characterised in that** said interspace has an inlet for connection to a bottle or bag of fluid.

4. A disposable active humidifier according to any one of the preceding claims, **characterised in that** said diffuser has inclined rings which are adapted to divert the stream toward said hydrophobic membrane.

## Patentansprüche

1. Wegwerfbarer aktiver Luftbefeuchter für die mechanische Beatmung eines Patienten, wobei der Luftbefeuchter eine Kassette aufweist, die eine Befeuchtungskammer bildet, die durch einen Einlass und einen Auslass begrenzt ist, wobei die Kassette dazu ausgelegt ist, in einen Beatmungskreislauf eingeschaltet zu werden, und wobei die Kassette einen Zwischenraum aufweist, der äußerlich durch eine Wärmeaustauschoberfläche begrenzt und innerlich durch eine hydrophobe Membran begrenzt ist, welche die Befeuchtungskammer umgibt, wobei eine Befeuchtungsflüssigkeit so in den Zwischenraum einführbar ist, dass die Befeuchtungsflüssigkeit, nachdem sie einmal in die Kassette eingeführt wurde, in der Kassette eingeschlossen bleibt, wenn sie nicht durch die hydrophobe Membran hindurch hieraus verdunstet, wobei die Kassette äußerlich mit der Wärmeaustauschoberfläche in der Form eines äußeren röhrenförmigen Gehäuses ausgestattet ist, das in der Verwendung als eine Schnittstelle zu einem Heizelement zum Beheizen der Befeuchtungsflüssigkeit im Luftbefeuchter dient, wobei die Befeuchtungskammer eine im Wesentlichen zylindrische Form hat und in der Verwendung einen Abschnitt des Beatmungskreislaufs bildet, **dadurch gekennzeichnet, dass** der Auslass und der Einlass durch entsprechende Deckel gebildet werden, welche den Zwischenraum und die Befeuchtungskammer axial begrenzen.

2. Wegwerfbarer aktiver Luftbefeuchter nach Anspruch 1, **dadurch gekennzeichnet, dass** er axial innerhalb der Befeuchtungskammer einen Druckluftbelüfter aufweist, der dazu ausgelegt ist, den Luftstrom des Beatmungskreislaufs gegen die hydrophobe Membran zu richten.

3. Wegwerfbarer aktiver Luftbefeuchter nach einem der vorhergehenden Ansprüche, **dadurch gekennzeichnet, dass** der Zwischenraum einen Einlass zur Verbindung mit einer Flasche oder einem Beutel mit Flüssigkeit hat.

4. Wegwerfbarer aktiver Luftbefeuchter nach einem der vorhergehenden Ansprüche, **dadurch gekennzeichnet, dass** der Druckluftbelüfter geneigte Ringe aufweist, die dazu ausgelegt sind, den Strom zur hydrophoben Membran hin abzulenken.

## Revendications

1. Humidificateur actif jetable pour la ventilation mécanique d'un patient, l'humidificateur comprenant une cartouche qui forme une chambre d'humidification qui est délimitée par une entrée et par une sortie, la cartouche étant adaptée pour être intercalée dans un circuit de ventilation, et ladite cartouche comportant un espacement qui est délimité à l'extérieur par une surface d'échange thermique et délimité à l'intérieur par une membrane hydrophobe qui entoure ladite chambre d'humidification, où un fluide d'humidification peut être introduit dans ledit espacement de telle sorte que le fluide d'humidification, une fois introduit dans la cartouche, demeure confiné à l'intérieur de la cartouche à moins d'une vaporisation à partir de celle-ci à travers la membrane hydrophobe, la cartouche étant prévue à l'extérieur avec ladite surface d'échange thermique sous la forme d'un boîtier tubulaire extérieur, qui, en utilisation, agit comme une interface avec un élément chauffant destiné à chauffer le fluide d'humidification à l'intérieur de l'humidificateur, où ladite chambre d'humidification présente une forme sensiblement cylindrique et, en utilisation, forme un segment dudit circuit de ventilation, **caractérisé en ce que** ladite sortie et ladite entrée sont formées par des bouchons respectifs qui délimitent axialement ledit espacement et ladite chambre d'humidification.

2. Humidificateur actif jetable selon la revendication 1, **caractérisé en ce qu'**il comprend axialement, à l'intérieur de ladite chambre d'humidification, un diffuseur qui est conçu pour diriger le flux d'air du circuit de ventilation contre ladite membrane hydrophobe.

3. Humidificateur actif jetable selon l'une quelconque des revendications précédentes, **caractérisé en ce que** ledit espacement présente une entrée pour une liaison avec un flacon ou une poche de fluide.

4. Humidificateur actif jetable selon l'une quelconque des revendications précédentes, **caractérisé en ce que** ledit diffuseur comporte des anneaux inclinés qui sont conçus pour dévier le flux vers ladite membrane hydrophobe.
